# EUROPÄISCHE PATENTANMELDUNG

(11) **EP 4 793 264 A1**
(43) Veröffentlichungstag der Anmeldung: **19.08.2026**
(21) Anmeldenummer: 25158452.0
(22) Anmeldetag: 18.02.2025
(51) Int. Cl.: C07C 45/50, C07F 9/6574, C07F 15/00

(54) **BISPHOSPHITE MIT MINDESTENS EINEM 1,1'-BI-2-NAPHTHOL-FLÜGELBAUSTEIN**

(71) Anmelder: Evonik Oxeno GmbH & Co. KG, 45772 Marl (DE)
(72) Erfinder: BÜKER, Julia, 44803 Bochum (DE); SALE, Anna Chiara, 40474 Düsseldorf (DE); FRANKE, Robert, 45772 Marl (DE); FRIDAG, Dirk, 45721 Haltern am See (DE); KUCMIERCZYK, Peter, 44628 Herne (DE); MARCOVIC, Ana, 45721 Haltern am See (DE)
(74) Vertreter: Evonik Patent Association

(57) **Zusammenfassung**

Bisphosphite mit mindestens einem 1,1'-Bi-2-naphthol-Flügelbaustein und deren Verwendung in der Hydroformylierung.

## Beschreibung

Die Erfindung betrifft Bisphosphite mit mindestens einem 1,1'-Bi-2-naphthol-Flügelbaustein und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die technische Aufgabe der Erfindung ist die Bereitstellung neuer Liganden, welche in der Hydroformylierung von Olefinen eine gute n/iso-Selektivität aufweisen.

Bei der Hydroformylierung gibt es die n/iso-Selektivität: das Verhältnis von linearem Aldehyd (= n) zu verzweigtem Aldehyd (= iso). Hierbei bedeutet die Selektivität bezüglich des n-Aldehyden, dass diese Menge an linearem Produkt gebildet wurde. Die restlichen Prozente entsprechen dann dem verzweigten Isomer. Bei einer Regioselektivität von 50% entstehen also n-Aldehyd und iso-Aldehyd zu gleichen Teilen.

Die Aufgabe wird gelöst durch eine Verbindung gemäß Anspruch 1.

Verbindung gemäß der Formel (I) oder (II): 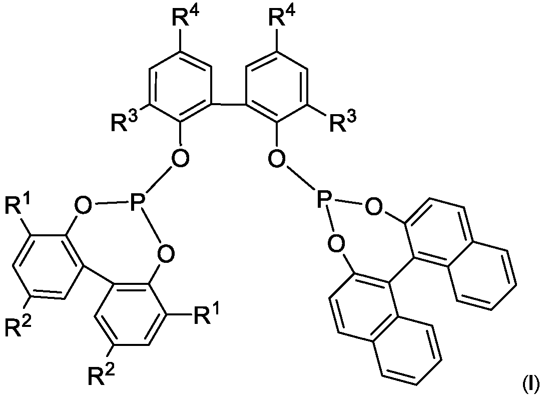 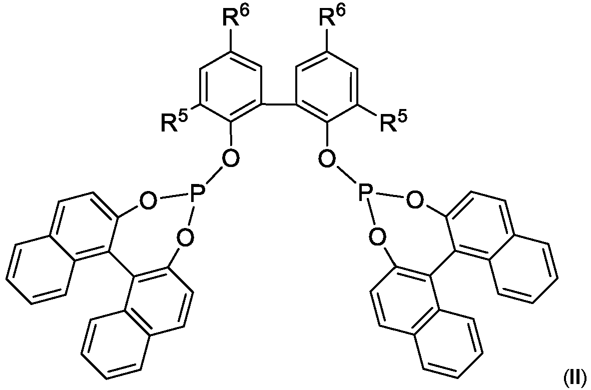 wobei
R¹, R², R³, R⁴, R⁵, R⁶ ausgewählt sind aus: -(C₁-C₁₂)-Alkyl, -O-(C₁-C₁₂)-Alkyl.

In einer Ausführungsform sind R¹, R², R³, R⁴, R⁵, R⁶ ausgewählt aus: -(C₁-C₆)-Alkyl, -O-(C₁-C₆)-Alkyl.

In einer Ausführungsform sind R¹, R², R³, R⁴, R⁵, R⁶ ausgewählt aus: -CH₃, -^{t}Bu, -OMe.

In einer Ausführungsform steht R⁴ für -OMe.

In einer Ausführungsform sind R¹, R³ ausgewählt aus: -CH₃, -^{t}Bu.

In einer Ausführungsform stehen R⁵, R⁶ für -CH₃.

In einer Ausführungsform stehen R¹, R² für -CH₃.

In einer Ausführungsform weist die Verbindung eine der Strukturen (1), (2) oder (3) auf:

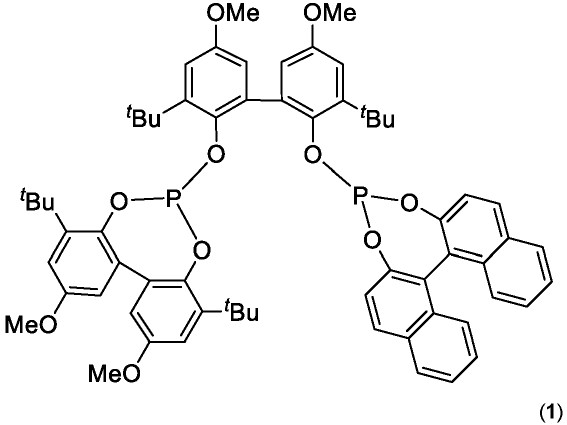

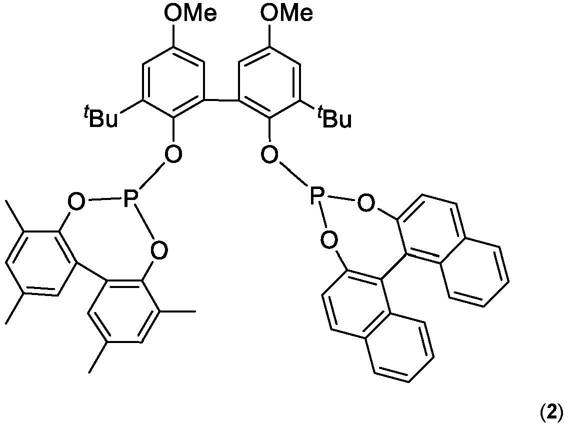

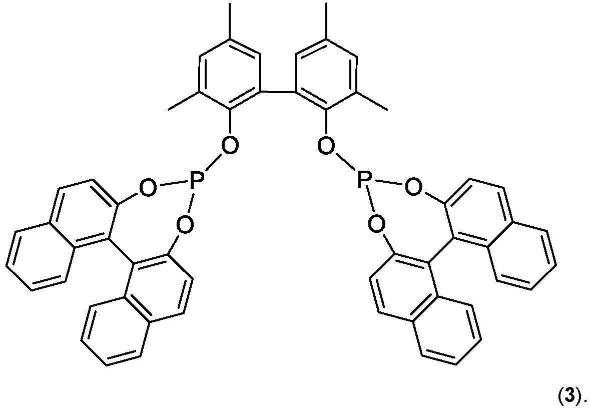

In einer Ausführungsform weist die Verbindung die Struktur (1) auf:

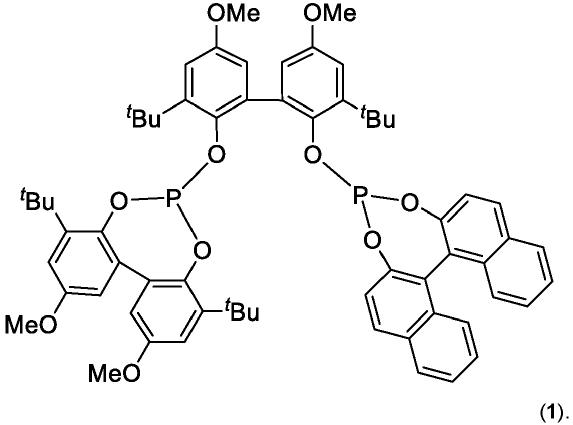

In einer Ausführungsform weist die Verbindung die Struktur (2) auf:

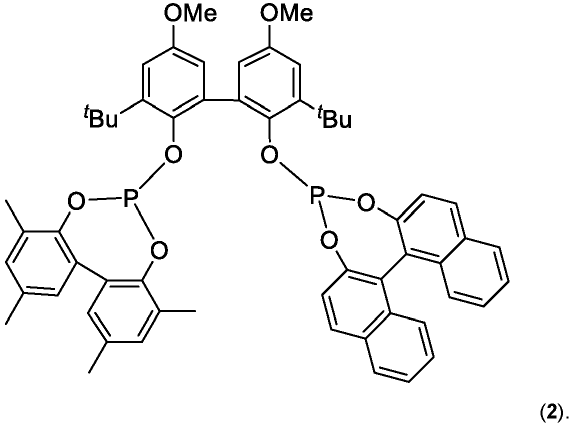

In einer Ausführungsform weist die Verbindung die Struktur (3) auf:

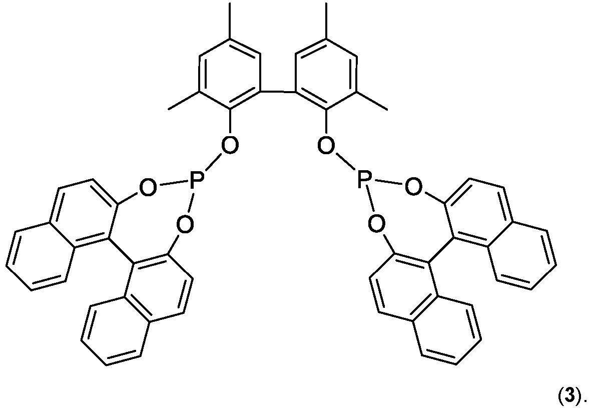

Neben der Verbindung als solche wird auch deren Verwendung zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Des Weiteren wird ein Verfahren beansprucht, in welchem die zuvor beschriebene Verbindung als Ligand eingesetzt wird.

Verfahren umfassend die Verfahrensschritte:
a) Vorlegen eines Olefins,
b) Zugabe einer zuvor beschriebenen Verbindung und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.

In einer bevorzugten Ausführungsform ist das Metall Rh.

Bevorzugt ist eine Temperatur von 80 °C bis 160 °C.

Besonders bevorzugt ist eine Temperatur von 100 °C bis 140 °C.

Im Folgenden soll die Erfindung anhand von Ausführungsbeispielen näher erläutert werden.

### Synthese (1)

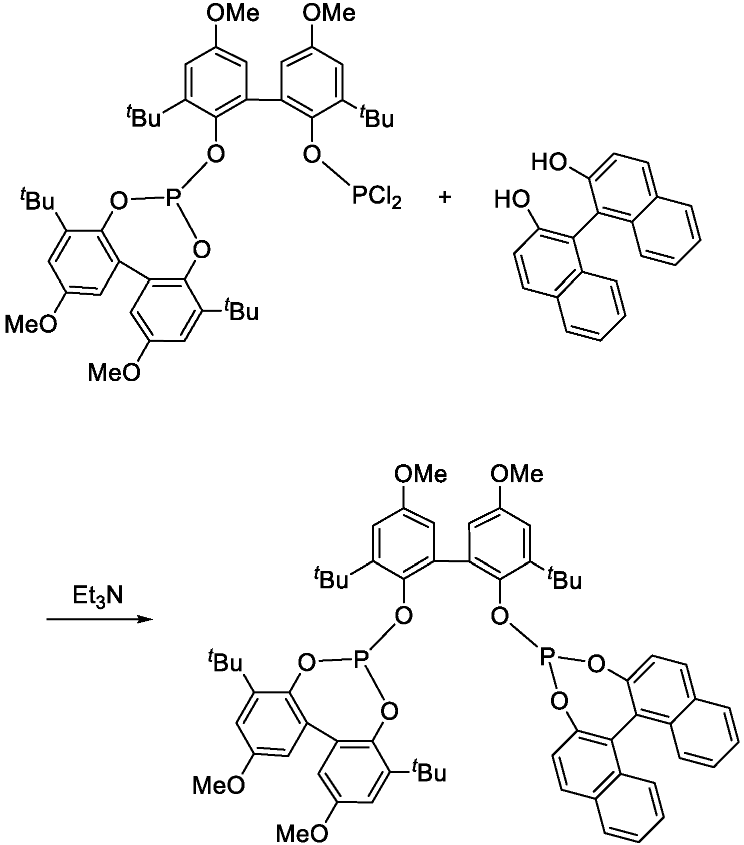

In der Glovebox werden 4.3 g (0.005 mol) Diorganodichlorophosphit abgewogen, ausgeschleust und 50 mL getrocknetes Toluol hinzugegeben.

In einem zweiten Schlenk werden 1.8 g (0.006 mol) Binol abgewogen und 15 Stunden mittels Ölpumpenvakuums getrocknet. Anschließend wird der Schlenk mit Argon geflutet. Das Binol wird in 30 mL getrocknetem Toluol gelöst und mit 3.6 mL (2.6 g, 0.025 mol) entgastem Triethylamin versetzt. Die Binol-Lösung wird langsam und stetig bei Raumtemperatur zur Chlorophosphit-Suspension gegeben. Die Reaktionslösung wird 15 Stunden bei Raumtemperatur gerührt.

Das entstandene Ammoniumhydrochlorid wird abgefrittet und mit 2 x 20 mL getrocknetem Toluol nachgewaschen. Das Filtrat wird anschließend mittels Ölpumpenvakuums bei 40 °C bis zur Trockene eingeengt.

Der Feststoff wird in 150 mL Isopropanol gerührt und bis zum Sieden erhitzt. Die Lösung wird heiß abfiltriert und langsam 15 Stunden abkühlen gelassen. Der auskristallisierte Feststoff wurde abgefrittet, mit Isopropanol gewaschen und mittels Ölpumpenvakuums getrocknet.
Ausbeute: 88.6 %

### Synthese (2)

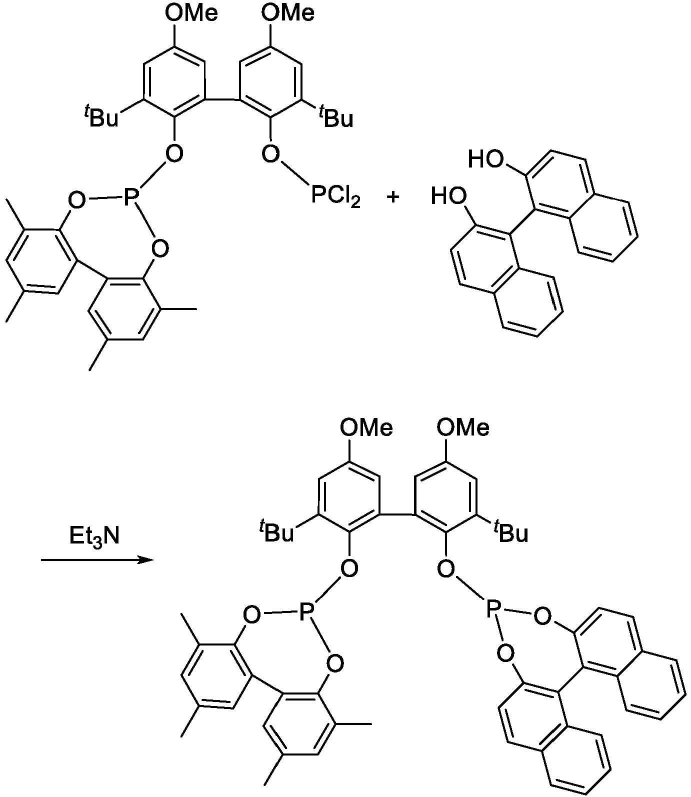

In der Glovebox werden 4.6 g (0.005 mol) Diorganodichlorophosphit abgewogen, ausgeschleust und 50 mL getrocknetes Toluol hinzugegeben.

In einem zweiten Schlenk werden 2.7 g (0.009 mol) Binol abgewogen und 15 Stunden mittels Ölpumpenvakuums getrocknet. Anschließend wird der Schlenk mit Argon geflutet. Das Binol wird in 40 mL getrocknetem Toluol gelöst und mit 5.4 mL (3.9 g, 0.0375 mol) entgastem Triethylamin versetzt. Die Binol-Lösung wurde langsam und stetig bei Raumtemperatur zur Chlorophosphit-Suspension gegeben. Die Reaktionslösung wurde 15 Stunden bei Raumtemperatur gerührt. Das entstandene Ammoniumhydrochlorid wird abgefrittet und mit 2 x 20 mL getrocknetem Toluol nachgewaschen. Das Filtrat wird anschließend mittels Ölpumpenvakuums bei 40 °C bis zur Trockene eingeengt.
Ausbeute: 58%

### Synthese (3)

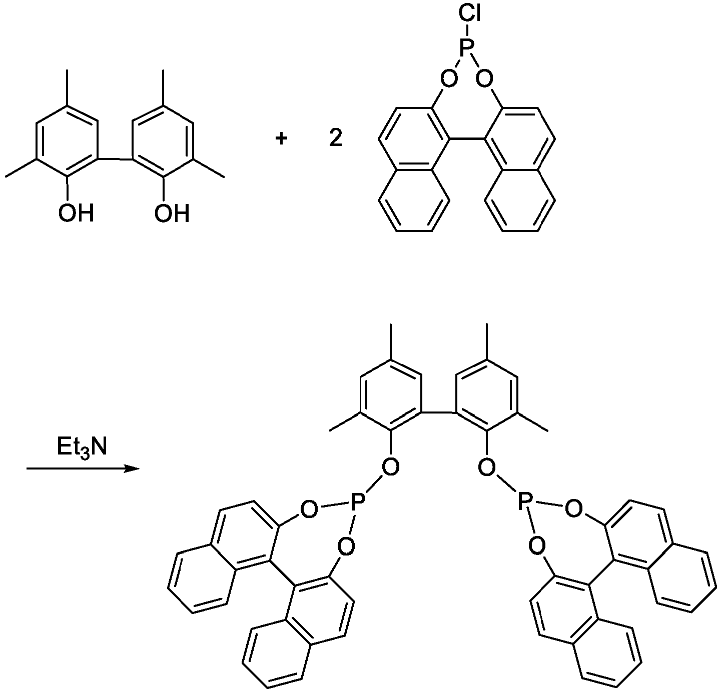

In der Glovebox werden 4.4 g (0.012 mol) Chlorophosphit abgewogen, ausgeschleust, und in 50 mL getrocknetem Toluol gelöst. Anschließend wird die Lösung auf -20 °C abgekühlt.

In einem zweiten Schlenk werden 1.2 g (0.005 mol) Biphenol abgewogen und 15 Stunden mittels Ölpumpenvakuums getrocknet. Anschließend wird der Schlenk mit Argon geflutet. Das Biphenol wird in 30 mL getrocknetem Toluol gelöst und mit 3.6 mL (2.6 g, 0.025 mol) entgastem Triethylamin versetzt. Die Biphenol-Lösung wird langsam und stetig bei -20 °C zur Chlorophosphit-Suspension gegeben. Die Reaktionslösung wird 15 Stunden bei Raumtemperatur gerührt. Das entstandene Ammoniumhydrochlorid wird abgefrittet und mit 2 x 20 mL getrocknetem Toluol gewaschen. Das Filtrat wird anschließend mittels Ölpumpenvakuums bei 40 °C bis zur Trockene eingeengt.

Der erhaltene gelbe Feststoff wird 15 Stunden in 100 mL getrocknetem Acetonitril bei Raumtemperatur gerührt. Am nächsten Tag wird der Feststoff abgefrittet, mit Acetonitril gewaschen und unter Ölpumpenvakuum getrocknet. Die Prozedur wird ein weiteres Mal wiederholt.
Ausbeute: 88,7%

### Synthese (4) (Vergleichsligand)

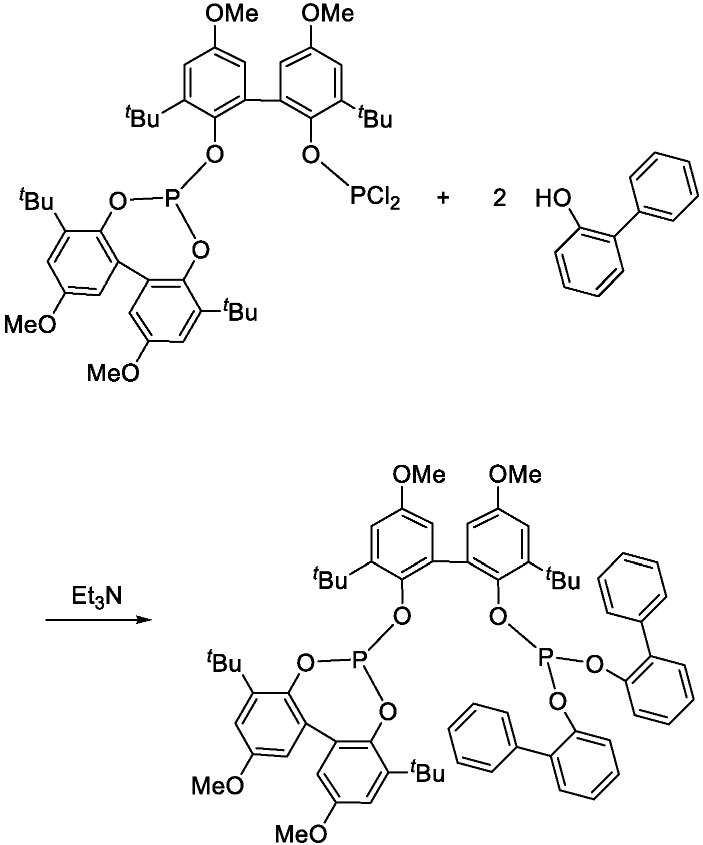

In der Glovebox wurde in einem 250 mL Schlenk 4.2 g (0.005 mol) Diorganodichlorophosphit abgewogen, ausgeschleust und 50 mL getrocknetes Toluol hinzugegeben.

In einem 100 mL Schlenk wurden 2.15 g (0.013 mol) 2-Phenylphenol abgewogen und über 15 Stunden mittels Ölpumpenvakuums getrocknet. Anschließend wurde der Schlenk mit Argon geflutet und das 2-Phenylphenol in 30 mL getrocknetem Toluol gelöst und 3.6 mL (2.6 g, 0.025 mol) entgastes Triethylamin hinzugegeben.

Die Phenylphenol-Lösung wurde langsam und stetig bei Raumtemperatur zur Chlorophosphit-Suspension gegeben. Die Reaktionslösung wurde über 15 Stunden bei Raumtemperatur gerührt. Es wurden zusätzlich 1.1 g (0.006 mol) 2-Phenylphenol in 10 mL getrocknetem Toluol und 1.8 mL (0.013 mol) Triethylamin gelöst und diese Lösung wurde bei Raumtemperatur langsam zur Reaktionslösung zugetropft. Die Reaktionslösung wurde auf 50 °C erhitzt und bei dieser Temperatur weitergerührt. Es wurden zusätzlich 1.1 g (0.006 mol) 2-Phenylphenol in 10 mL getrocknetem Toluol und 1.8 mL (0.013 mol) Triethylamin gelöst und diese Lösung wurde bei Raumtemperatur langsam zur Reaktionslösung zugetropft. Das entstandene Ammoniumhydrochlorid wurde abgefrittet, mit 2 x 20 mL getrocknetem Toluol nachgewaschen und das Filtrat wurde anschließend mittels Ölpumpenvakuums bei 40 °C bis zur Trockene eingeengt. Der Feststoff wurde in 100 mL getrocknetem Methanol bei Raumtemperatur gerührt, abgefrittet, mit 2x50 mL Methanol gewaschen und nach dem Trocknen in die Glovebox eingeschleust. Der Feststoff wurde in Acetonitril gerührt und zum Sieden erhitzt. Dann wurde so viel Acetonitril hinzugeben, bis eine klare Lösung entstand. Die Heizung und der Rührer wurden abgestellt, so dass der Feststoff langsam auskristallisieren konnte. Das erhaltene Filtrat wurde über 15 Stunden in den Kühlschrank gestellt. Über 15 Stunden ist ein hellbrauner Feststoff auskristallisiert, welcher abfiltriert wurde, mit kaltem Acetonitril nachgewaschen und anschließend getrocknet wurde.
Ausbeute: 92.8%

### Katalyseversuche

Es wird unter Argon-Atmosphäre gearbeitet. Als Substrat eingesetzt wurde n-Octen (Oxeno GmbH, Octenisomerengemisch aus 1-Octen: 3 %; cis+trans-2-Octen: 49 %; cis+trans-3-Octen: 29 %; cis+trans-4-Octen: 16 %; gerüstisomere Octene: 3 %).

Es wurde eine Stammlösung vorab unter Argonatmosphäre vorbereitet. Hierfür wurden 0,0127 g Rh(acac)(CO)₂ und die entsprechende Menge an Phosphitverbindung (Lig:Rh = 5:1) eingewogen und mit 48,0 ml Toluol aufgefüllt. Die Vials wurden im Reaktor platziert und dieser verschlossen. Es wurde dreimal mit Argon und dreimal mit Synthesegas (Linde; H₂ (99,999 %) : CO (99,997%) = 1:1) gespült. Nach erfolgter Druckprüfung wurde der Autoklav bei einem Gesamtdruck von 10 bar unter Rühren auf die angestrebte Temperatur von 120°C aufgeheizt. Nach Erreichen der Reaktionstemperatur wurde der Synthesegasdruck auf 17 bar erhöht und das Substrat mittels HPLC-Pumpe mit je 2 mL zum Reaktionsstart zudosiert. Daraus ergibt sich eine Rh-Konzentration von 100 ppm. Nach 1 h bei konstantem Druck wurde eine Probe gezogen und unverdünnt gaschromatographisch analysiert.

### Reaktionsbedingungen:

[Rh]: 100 ppm, p: 17 bar, T: 120 °C; t: 1 h

Die Ergebnisse der Katalyseversuche sind in der nachfolgenden Tabelle aufgelistet.

**Tabelle**

| Ligand | n/iso-Selektivität [%] |
|---|---|
| **(1)*** | 83 |
| **(2)*** | 80 |
| **(3)*** | 85 |
| **(4)** | 34 |

| | |
|---|---|
| * erfindungsgemäßes Ausführungsbeispiel | |

Die durchgeführten Versuche belegen, dass die gestellte Aufgabe durch die erfindungsgemäßen Verbindungen gelöst wird.

## Patentansprüche

1. Verbindung gemäß der Formel (I) oder (II): 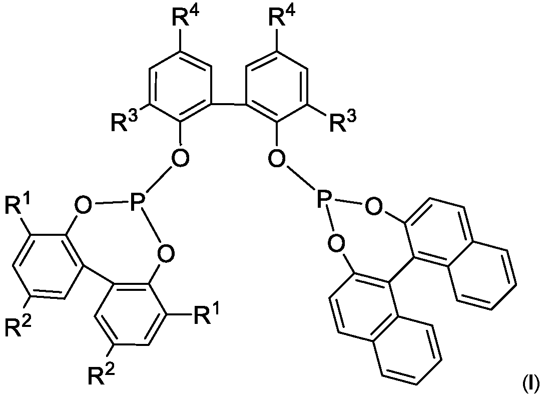 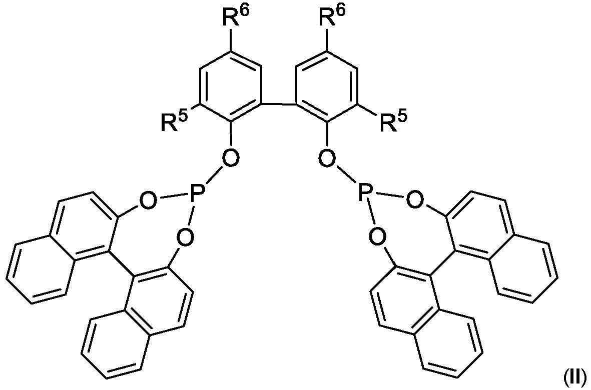 wobei
R¹, R², R³, R⁴, R⁵, R⁶ ausgewählt sind aus: -(C₁-C₁₂)-Alkyl, -O-(C₁-C₁₂)-Alkyl.

2. Verbindung nach Anspruch 1,
wobei R¹, R², R³, R⁴, R⁵, R⁶ ausgewählt sind aus: -(C₁-C₆)-Alkyl, -O-(C₁-C₆)-Alkyl.

3. Verbindung nach einem der Ansprüche 1 bis 2,
wobei R¹, R², R³, R⁴, R⁵, R⁶ ausgewählt sind aus: -CH₃, -^{t}Bu, -OMe.

4. Verbindung nach einem der Ansprüche 1 bis 3,
wobei R⁴ für -OMe steht.

5. Verbindung nach einem der Ansprüche 1 bis 4,
wobei R¹, R³ ausgewählt sind aus: -CH₃, -^{t}Bu.

6. Verbindung nach einem der Ansprüche 1 bis 5,
wobei R⁵, R⁶ für -CH₃ stehen.

7. Verbindung nach einem der Ansprüche 1 bis 6,
wobei R¹, R² für -CH₃ stehen.

8. Verbindung nach einem der Ansprüche 1 bis 7,
wobei die Verbindung eine der Strukturen (1), (2) oder (3) aufweist:

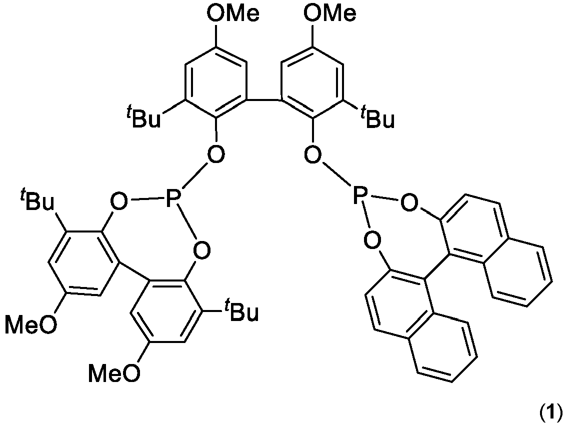

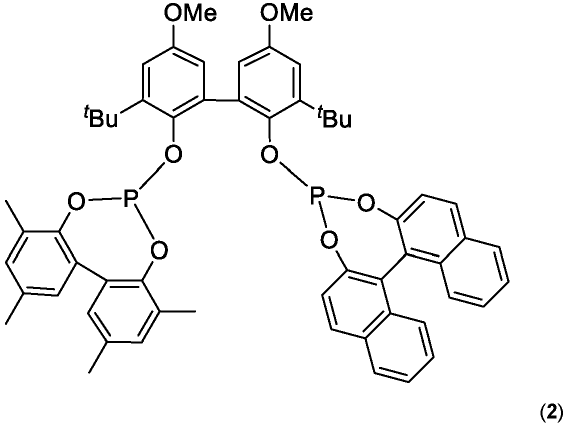

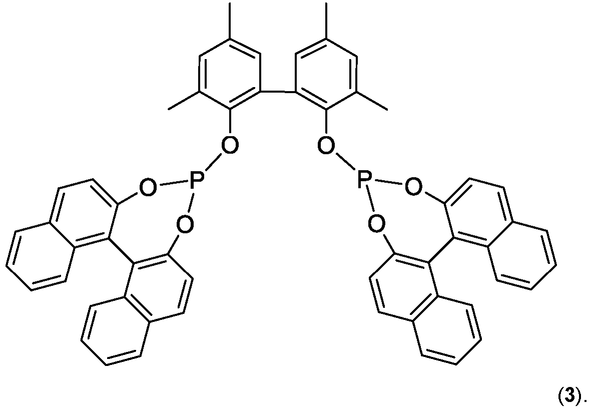

9. Verbindung nach einem der Ansprüche 1 bis 8,
wobei die Verbindung die Struktur (1) aufweist:

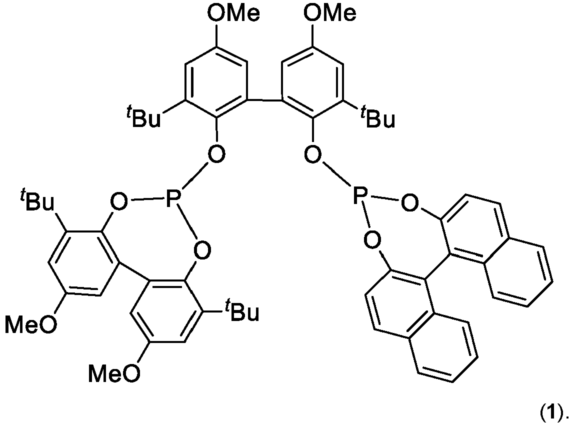

10. Verbindung nach einem der Ansprüche 1 bis 8,
wobei die Verbindung die Struktur (2) aufweist:

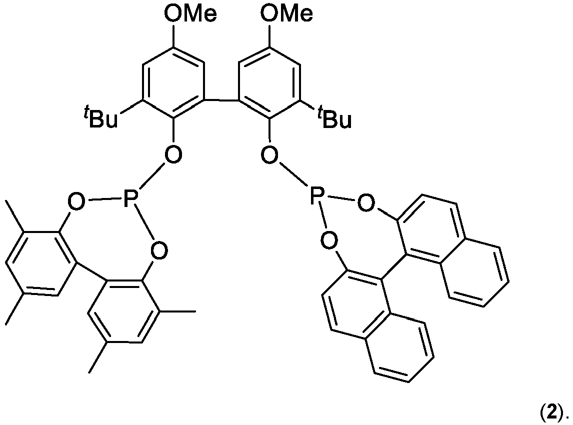

11. Verbindung nach einem der Ansprüche 1 bis 8,
wobei die Verbindung die Struktur (3) aufweist:

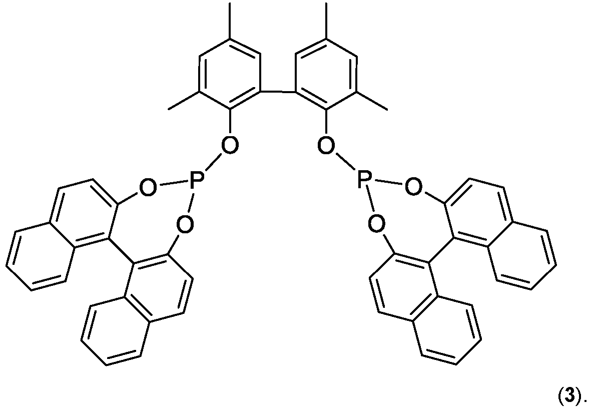

12. Verwendung einer Verbindung nach Anspruch 1 bis 11,
in einem Ligand-Metall-Komplex zur Katalyse einer Hydroformylierungsreaktion.

13. Verfahren umfassend die Verfahrensschritte:
a) Vorlegen eines Olefins,
b) Zugabe einer Verbindung nach einem der Ansprüche 1 bis 11 und einer Substanz, welche ein Metall ausgewählt aus: Rh, Ru, Co, Ir aufweist,
c) Zuführen von H₂ und CO,
d) Erwärmen des Reaktionsgemisches aus a) bis c), wobei das Olefin zu einem Aldehyd umgesetzt wird.
